# EUROPEAN PATENT SPECIFICATION

(11) **EP 0 334 440 B1**
(45) Date of publication and mention of the grant of the patent: **18.05.1994**
(21) Application number: 89200682.6
(22) Date of filing: 17.03.1989
(51) Int. Cl.: A61M 16/00

(54) **Respirating apparatus for patients**
Beatmungsgerät für Patienten
Appareil respiratoire pour patients

(30) Priority: 23.03.1988 NL 8800718
(43) Date of publication of application: 27.09.1989
(73) Proprietor: PHYSIO BV, 1382 JR Weesp (NL)
(72) Inventor: Westerkamp, Bart, Alkmaar (NL); van Dijk, Geert, Maarssen (NL)
(74) Representative: Koomen, M.J.I.

(56) References cited:
- NL-A- 6 906 142
- US-A- 4 620 536

## Description

The invention relates to a respirating apparatus for patients, more in particular for anaesthesia, formed by a housing with means for the forming and administering of the respiratory gas, and with regulating- and control-equipment (monitor) belonging to it.

Such an apparatus is known.

In this apparatus, the housing in general has the shape of a rectangular parallelepiped, and thus as a rule a rectangular horizontal section.
This shape of housing has the disadvantage, that it takes up a relatively large amount of space at the operating table, which could be used more suitably by the surgical staff.
Further, the shape of the known apparatus has the disadvantage, that the respiratory tubes and suction tubes have to be relatively long, because it is difficult or impossible to bring the apparatus with the patient-connections up close to the patient.

US-A-4620536 discloses a modular tray assembly for a respiratory apparatus corresponding to the preamble of Claim 1.

The invention intends to obviate these drawbacks of the known apparatus.

The apparatus according to the invention is thereto characterized, in that the housing substantially has the shape of a prism or a cylinder sector with three sidefaces connected to each other over edge-lines, parallel to one another, while in or near a first corner- or nose part, enclosed by two sidefaces, the connecting means for the patient have been installed.

Due to the point shape or triangular shape of the apparatus according to the invention, the patient-connections can be brought close to the patient, without negatively influencing the accessibility of the surgical staff to the operating table.

While the first, preferably the most pointy corner or nose part contains the patient-connections, according to a further working-out of the apparatus according to the invention, both other corner parts are turnable around vertical rotation axes towards the first corner part up to against the sidefaces.

Due to this measure, it is possible with the turning back of both these other corner parts to reduce the complete width of the backside of the apparatus, which may be of importance for the transportation of the apparatus through narrow passages, such as doorways and the like.

In a favourable embodiment of the apparatus according to the invention, the angle, under which the sidefaces enclose the first corner part or nose part, is between the 30 degrees and the 60 degrees.

In a preferable embodiment of the invented apparatus, the regulating- and control-equipment has been installed on the housing as a unit, rotatable around a vertical axis, in such a way, that the apparatus is operatable by an anaesthetist from each of the three sidefaces.

By this measure the anaesthetist can perform his work, without hindering the surgical staff in their movements.

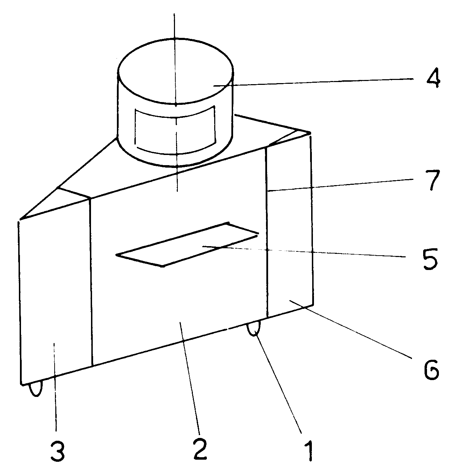

The invention will now be explained more closely with reference to the drawing of an embodiment by way of example.

As shown schematically in the drawing, the apparatus according to this example of an embodiment has been provided with a housing 2 in the shape of a prism, movable on swivel castors 1, with a sharp nose part or a first corner part 3, that has been provided with the connections for the patient, such as respiratory tubes, the oxigenation set, the suction tubes, etc.

On the housing the regulating- and control-equipment has been installed, which is rotatable in respect to the housing, so that the anaesthetist may operate the apparatus from the backside as well as from the sidefaces which border the nose part 3, and thus may take in a position which is the least hindering to the surgical staff.

Each sideface of the housing has been provided with a writing plate 5, which can be drawn from out of the housing.
Both the back corner parts 6 are turnable to the front around the vertical axes 7, causing the width of the backside of the housing to be reduced, which may be of importance for the transportation of the apparatus through narrow passages.

Furthermore, these rotatable back corner parts 6, which may be turned away or out, may serve as storage space for medicine, accessories, bandages, etc.

## Claims

1. Respirating apparatus for patients, more in particular for anaesthesia, formed by a housing (2) with an apparatus for the forming and administering of the respiratory gas and with regulating- and control-equipment belonging to it, characterized in that the housing has the shape of a prism or a cylinder sector with three sidefaces connected to each other over edge-lines, parallel to one another, while in or near a first corner part (3), enclosed by two sidefaces, the connecting means for the patient have been installed.

2. Apparatus according to claim 1, characterized in that both the other corner parts (6) may be turned towards the first corner part around vertical rotation axes (7), up to against the sidelevels.

3. Apparatus according to claim 1 or 2, characterized in that the angle, under which the sidefaces enclose the first corner part, is smaller than those of both the other corner parts.

4. Apparatus according to claim 3, characterized in that the angle of the first corner part is between 30 and 60 degree.

5. Apparatus according to any of the preceding claims, characterized in that the regulating- and control-equipment has been installed on the housing as a unit (4), rotatable around a vertical axis, in such a way, that the apparatus is operatable from each of the three sides.

6. Apparatus according to any of the preceding claims, characterized in that in each of the three sidefaces at least a writing plate (5) has been installed which can be drawn out of the housing.

7. Apparatus according to claim 2, characterized in that the turnable corner parts have been carried out as storage compartiments.

## Patentansprüche

1. Beatmungsgerät für Patienten, das insbesondere für die Anästhesie bestimmt ist und von einem Gehäuse (2) gebildet wird, das mit einem Gerät zur Bildung und Verabreichung des Atmungsgases sowie mit einer dazugehörigen Regulier- und Steuervorrichtung versehen ist, **dadurch gekennzeichnet, dass** das Gehäuse (2) die Form eines Prismas oder eines Zylindersektors besitzt, der drei Seitenflächen aufweist, die über zueinander parallele Randlinien miteinander verbunden sind, während die Verbindungsmittel zum Patienten im oder nahe dem ersten Eckteil (3) angeordnet sind und von zwei Seitenflächen eingeschlossen werden.

2. Beatmungsgerät nach Anspruch 1, **dadurch gekennzeichnet dass** die beiden andern Eckteile (6) um senkrechte Drehachsen (7) bis zu den Seitenflächen gegen den ersten Eckteil (3) geschwenkt werden können.

3. Beatmungsgerät nach Anspruch 1 oder 2, **dadurch gekennzeichnet, dass** der Winkel, unter dem die Seitenflächen den ersten Eckteil (3) einschliessen, kleiner ist als diejenigen der beiden andern Eckteile (6).

4. Beatmungsgerät nach Anspruch 3, **dadurch gekennzeichnet, dass** der Winkel des ersten Eckteils (3) zwischen 30 und 60 Grad beträgt.

5. Beatmungsgerät nach einem der Ansprüche 1 bis 4, **dadurch gekennzeichnet, dass** die Regulier- und Steuervorrichtung als Einheit auf dem Gehäuse (2) angeordnet und derart um eine vertikale Achse drehbar ist, dass das Gerät von jeder der drei Seitenflächen aus bedient werden kann.

6. Beatmungsgerät nach einem der Ansprüche 1 bis 5, **dadurch gekennzeichnet, dass** auf jeder der drei Seitenflächen mindestens eine Schreibplatte (5) angeordnet ist, die aus dem Gehäuse (2) herausgezogen werden kann.

7. Beatmungsgerät nach Anspruch 2, **dadurch gekennzeichnet, dass** die schwenkbaren Eckteile (6) als Aufbewahrungsabteile ausgebildet sind.

## Revendications

1. Appareil respiratoire pour des patients, plus particulièrement pour l'anesthésie, comportant un boîtier (2) pourvu d'un appareillage pour produire et administrer le gaz respiratoire et d'un équipement de régulation et de contrôle qui en fait partie, caractérisé en ce que le boîtier a la forme d'un prisme ou d'un secteur de cylindre à trois faces latérales reliées l'une à l'autre par des bords rectilignes parallèles entre eux, tandis que les moyens de connexion pour le patient sont disposés dans un premier coin (3) du boîtier, compris entre deux faces latérales, ou à proximité de ce premier coin.

2. Appareil selon la revendication 1, caractérisé en ce que les deux autres coins du boîtier peuvent tourner et se rabattre vers le premier coin, en pivotant autour d'axes verticaux jusqu'à venir en appui contre les faces latérales.

3. Appareil selon la revendication 1 ou 2, caractérisé en ce que l'angle formé par les faces latérales à l'endroit du premier coin est plus petit que ceux des autres coins.

4. Appareil selon la revendication 3, caractérisé en ce que l'angle du premier coin est compris entre 30 et 60 degrés.

5. Appareil selon l'une des revendications 1 à 4, caractérisé en ce que l'équipement de régulation et de contrôle est monté sur le boîtier, sous forme d'un ensemble unitaire pouvant tourner autour d'un axe vertical, pour permettre la mise en oeuvre de cet appareil à partir de l'une des trois faces latérales.

6. Appareil selon l'une des revendications 1 à 5, caractérisé en ce qu'il comporte à l'endroit de chacune des trois faces latérales au moins une tablette pour écrire (5) pouvant être tirée hors du boîtier.

7. Appareil selon la revendication 2, caractérisé en ce que les coins qui peuvent tourner sont aménagés en compartiments de rangement.
